# FASCICULE DE BREVET EUROPEEN

(11) **EP 3 851 143 B1**
(45) Date de publication et mention de la délivrance du brevet: **12.10.2022**
(21) Numéro de dépôt: 20213786.5
(22) Date de dépôt: 14.12.2020
(51) Int. Cl.: A61M 16/00, A61M 16/12

(54) **VENTILATEUR MÉDICAL AVEC AFFICHAGE SIMULTANÉ DE DIFFÉRENTES PRESSIONS**
MEDIZINISCHES BEATMUNGSGERÄT MIT GLEICHZEITIGER ANZEIGE VERSCHIEDENER DRÜCKE
MEDICAL VENTILATOR WITH SIMULTANEOUS DISPLAY OF DIFFERENT PRESSURES

(30) Priorité: 14.01.2020 FR 2000317
(43) Date de publication de la demande: 21.07.2021
(73) Titulaire: Air Liquide Medical Systems, 92160 Antony (FR)
(72) Inventeur: ANDRE DIAS, Sofia, 92160 Antony (FR); GERMANI, Damien, 92320 Chatillon (FR); FISHMAN, Clara, 92160 Antony (FR); CAPIROSSI, Jean-Luc, 92160 Antony (FR)
(74) Mandataire: Air Liquide

(56) Documents cités:
- WO-A1-2017/149532
- WO-A1-2019/240634
- US-A1- 2017 014 587

## Description

L'invention concerne un appareil de ventilation médical pour fournir un gaz respiratoire à un patient comprenant un afficheur graphique configuré pour afficher différentes pressions de manière à assister le personnel soignant pendant la ventilation du patient et ainsi éviter les barotraumatismes, les hypoxies ou autres.

Il est courant d'utiliser un appareil de ventilation, aussi appelé ventilateur médical ou appareil d'assistance respiratoire, pour fournir un gaz respiratoire, tel de l'air enrichi ou non en oxygène, à un patient souffrant de troubles respiratoires, telle la BPCO ou autre. Le gaz peut être délivré par une micro-soufflante, aussi appelée turbine ou compresseur.

Un ventilateur médical peut aussi comprendre un afficheur, tel un écran d'affichage, sur lequel sont affichées des données utiles au personnel soignant, typiquement des courbes ou graphiques de monitorage respiratoire du patient, pendant qu'il est relié au ventilateur et inhale le gaz respiratoire fourni par le ventilateur.

Les différentes courbes de suivi et autres paramètres respiratoires, par exemple des courbes de pression et de débit, s'affichent indépendamment les unes des autres sur l'afficheur du ventilateur.

Le problème qui en résulte est que le personnel soignant doit alors réfléchir par lui-même à la corrélation existant entre les différentes courbes et paramètres, ce qui peut conduire à des erreurs d'interprétation pouvant conduire à de mauvais réglages subséquents du ventilateur, donc à une délivrance de gaz au patient moins ou peu sécurisée.

Par ailleurs, pour permettre un affichage lisible par le personnel soignant, il est nécessaire de prévoir un afficheur, typiquement un écran digital, de grande taille, ce qui augmente l'encombrement général du ventilateur et son poids. Or, ceci peut être un inconvénient important pour le personnel soignant, lorsque le ventilateur doit servir dans un service d'intervention d'urgence, tel que SAMU, pompiers..., où il porté par les secouristes jusque sur le lieu de chaque intervention.

Pour éviter ou minimiser ces problèmes, certains ventilateurs comprennent des afficheurs configurés pour n'afficher que certaines courbes ou autres informations ventilatoires, ce qui permet de réduire la taille des afficheurs, i.e. écrans.

Toutefois, si cela permet de réduire l'encombrement des afficheurs (taille, poids...), un problème qui en découle est que le personnel soignant ne dispose pas alors de toutes les informations utiles pour opérer un soin vraiment efficace et/ou sécurisé du patient. La ventilation du patient peut s'en trouver alors négativement impactée.

Le problème qui se pose alors est de proposer un appareil de ventilation de patient, c'est-à-dire un ventilateur médical, amélioré qui permette d'afficher des informations utiles au personnel soignant sur une zone d'affichage réduite de manière à limiter l'encombrement de l'afficheur utilisé tout en procurant un maximum de données utiles, notamment des valeurs de pressions, au personnel soignant et ce, de manière déjà corrélée, c'est-à-dire sans qu'il n'ait à réfléchir pour réaliser une corrélation entre les différents paramètres affichés puisque la corrélation est opérée par le ventilateur lui-même.

Le document WO2019/240634 divulgue un appareil respiratoire.

La solution de l'invention porte alors sur un appareil de ventilation, c'est-à-dire un ventilateur médical, pour fournir un gaz respiratoire à un patient comprenant une micro-soufflante délivrant un gaz respiratoire, un circuit de gaz interne pour convoyer le gaz respiratoire, au moins un capteur de pression pour mesurer la pression du gaz, une interface graphique utilisateur (IGU) et une unité de commande électronique reliée électriquement à la micro-soufflante, au capteur de pression et à l'interface graphique utilisateur (IGU), ledit (au moins un) capteur de pression étant par ailleurs configuré pour mesurer et transmettre à l'unité de commande électronique, des signaux de mesure de pression, caractérisé en ce que :
- il comprend des moyens de réglage configurés pour permettre à un utilisateur de fixer (par exemple entrer) ou sélectionner (par exemple choisir):
   ∘ au moins une valeur de pression d'aide inspiratoire (AI) comprise entre 1 et 50 cmH₂O et
   ∘ au moins une valeur de pression expiratoire positive (PEP) comprise entre 0 et 50 cmH₂O,
- l'unité de commande électronique (12) est configurée pour calculer, fixer, sélectionner ou déterminer:
   ▪ une pression maximale (Pₘₐₓ) à partir des valeurs de pression d'aide inspiratoire (AI) et de pression expiratoire positive (PEP) fixées ou sélectionnées par l'utilisateur, avec Pₘₐₓ = AI + PEP + k, où k est une valeur de pression non-nulle préfixée comprise entre 1 et 20 cm H₂O, et
   ▪ une pression minimale (Pₘᵢₙ) comprise entre 1 et 10 cm H₂O, avec Pₘᵢₙ<AI+PEP,
- l'unité de commande électronique est configurée pour calculer ou déterminer, à partir des signaux de mesure de pression provenant du capteur de pression, au moins :
   ▪ une pression expiratoire (Pₑₓₚᵢ) du patient correspondant à la valeur de pression mesurée à la fin de chaque phase expiratoire du patient,
   ▪ une pression inspiratoire (Pᵢₙₛₚ) du patient correspondant à la valeur de pression mesurée à la fin de chaque phase inspiratoire du patient et
   une pression instantanée (Pᵢₙₛₜ) correspondant à la valeur de pression instantanée mesurée en continu,
- et l'interface graphique utilisateur est configurée pour afficher simultanément sur un graphique unique, au moins : la pression maximale (Pₘₐₓ), la pression minimale (Pₘᵢₙ), la pression expiratoire (Pₑₓₚᵢ), la pression inspiratoire (Pᵢₙₛₚ) et la pression instantanée (Pᵢₙₛₜ).

Dans le cadre de l'invention :
- un cycle ventilatoire est défini comme la période de temps correspondant aux phases inspiratoire et expiratoire du patient. Typiquement, un cycle respiratoire a une durée de l'ordre de 1 à 5 secondes, typiquement de l'ordre de 3 à 4 secondes chez les patients adultes. Toutefois, chez les nourrissons et les enfants, elle peut être de durée inférieure à 3 secondes, par exemple de l'ordre de 1 à 2 secondes.
- un patient est une personne humaine, i.e. un individu, quel que soit son âge (adulte, personne âgée, adolescent, enfant, nouveau-né...), souffrant d'un trouble respiratoire, par exemple une pathologie respiratoire de type SDRA, un BPCO en crise aiguë, ayant besoin d'une assistance respiratoire.
- les pressions sont exprimées en centimètres d'eau ou « cmH₂O ».

Selon le cas, l'appareil de ventilation de l'invention peut comprendre l'une ou plusieurs des caractéristiques techniques suivantes :
- la pression minimale (Pₘᵢₙ) est fixée à :
   ▪ 1 cmH₂O lorsque AI + PEP < 4 cmH₂O,
   ▪ 4 cmH₂O lorsque 4 cmH₂O ≤ Al + PEP ≤ 14 cmH₂O, et
   ▪ 10 cmH₂O lorsque AI + PEP > 14 cmH₂O.
- la valeur de pression k est comprise entre 5 et 15 cmH₂O, de préférence égale à environ 10 cmH₂O.
- la valeur de PEP est comprise entre 0 et 30 cmH₂O, de préférence entre 0 et 16 cmH₂O.
- la valeur d'aide inspiratoire (AI) est comprise entre 2 et 35 cm H₂O, de préférence entre 4 et 26 cmH₂O.
- la valeur d'aide inspiratoire (AI) est de préférence d'au moins 10 cmH₂O.
- l'IGU comprend un écran d'affichage, de préférence un écran numérique, i.e. digital.
- l'IGU est configurée pour afficher les niveaux et/ou valeurs de pression sur un graphique unique représentant un barre-graphe.
- le barre-graphe a une forme allongée, typiquement rectangulaire, telle une colonne.
- l'IGU est configurée pour afficher des repères visuels représentant des niveaux de pression.
- l'IGU est configurée pour afficher des caractères ou textes identifiant la nature des repères visuels.
- l'IGU est configurée pour afficher des valeurs numériques de pressions en regard des repères visuels.
- les valeurs d'AI et de PEP sont telles que : AI > PEP.
- les valeurs d'AI et de PEP sont réglées ou sélectionnées par le personnel soignant, par exemple un médecin, en fonction du patient, en particulier de sa pathologie, de l'état de ses poumons etc....
- la valeur de Pmin est telle que : Pmin < AI.
- la pression expiratoire (Pₑₓₚᵢ) correspond à la valeur de pression en fin d'expiration du patient qui est généralement la valeur de pression la plus haute du cycle respiratoire considéré.
- l'IGU comprend un écran d'affichage tactile.
- l'IGU comprend un écran d'affichage en noir et blanc ou en couleurs.
- l'IGU est configurée pour permettre de passer d'un affichage en noir et blanc à un affichage en couleurs, ou inverse, par exemple après appui sur une touche dédiée.
- l'IGU est configurée pour afficher un ou des niveaux et/ou valeurs de pression sur un graphique unique représentant un barre-graphe, de préférence un barre-graphe rectiligne ou longiligne.
- l'IGU est configurée pour afficher des repères visuels représentant des niveaux de pression.
- l'IGU est configurée pour afficher des alphanumériques, tels des caractères ou textes, identifiant la nature des repères visuels.
- l'IGU est configurée pour afficher des valeurs numériques de pressions en regard des repères visuels, notamment en regard des caractères ou textes identifiant la nature des repères visuels.
- optionnellement, l'IGU est configurée pour afficher les valeurs d'AI et/ou de PEP.
- l'unité de commande électronique pilote la micro-soufflante pour fournir le gaz respiratoire selon des cycles ventilatoires, en général successifs.
- l'unité de commande électronique comprend un microprocesseur, de préférence un microcontrôleur.
- l'unité de commande électronique comprend une carte électronique, de préférence le microprocesseur est porté par la carte électronique.
- le (au moins un) capteur de pression est configuré pour fournir à l'unité de commande électronique, au moins une valeur (i.e. signal) de pression inspiratoire instantanée (Pinst), de préférence plusieurs valeurs de pression inspiratoire instantanée (Pᵢₙₛₜ) successives.
- le capteur de pression est configuré pour déterminer, i.e. mesurer, la pression inspiratoire instantanée (Pᵢₙₛₚᵢ) en continu ou quasi-continu.
- la micro-soufflante comprend un moteur électrique, i.e. elle est motorisée.
- la micro-soufflante comprend un moteur électrique entraînant un axe-moteur rotatif portant une (ou plusieurs) roue à ailettes ou pales agencée dans un compartiment à roue au sein d'une volute.
- l'unité de commande électronique pilote la micro-soufflante motorisée (i.e. accélération et freinage ou décélération du moteur électrique) pour fournir le gaz respiratoire à débit et pression donnés, de préférence fixés ou sélectionnés par l'utilisateur.
- il comprend une alarme sonore et/ou visuelle, activée par l'unité de commande électronique en réponse à une détection par ladite unité de commande électronique d'une pression inspiratoire instantanée (Pᵢₙₛₚᵢ) en fin de phase inspiratoire telle que Pᵢₙₛₚᵢ < Pₘᵢₙ.
- l'IGU est configurée pour afficher sur le graphique unique, des repères visuels fixes correspondant à au moins une partie des pressions à afficher, c'est-à-dire non-mobiles.
- les repères visuels fixes (i.e. marquages) sont des tirets, flèches ou toute autre forme ou icone.
- l'IGU est configurée pour afficher sur le graphique unique, un repère visuel mobile correspond à la valeur variable de pression instantanée (Pᵢₙₛₜ). La pression instantanée (Pᵢₙₛₜ) fluctue en continu durant chaque cycle ventilatoire en fonction du niveau de pression délivrée par la micros-soufflante de l'appareil.
- le repère visuel mobile correspond à Pᵢₙₛₜ est ou comprend une barre horizontale.
- le repère visuel mobile correspond à Pᵢₙₛₜ délimite une zone grisée, par exemple une zone colorée ou analogue, représentative de la Pinst mesurée.
- l'IGU est configurée pour afficher un graphique unique de type barre-graphe, de préférence un barre-graphe rectiligne, c'est-à-dire ayant la forme d'un rectangle vertical allongé (i.e. une colonne).
- l'IGU est configurée pour afficher un graphique unique de type barre-graphe avec une zone colorée dont la surface colorée varie en fonction de la valeur de Pinst, de manière à afficher, de préférence en continu, sur le graphique unique, les variations ou évolutions instantanées au fil du temps de la pression instantanée (Pᵢₙₛₜ).
- il comprend des caractères ou textes identifiant la nature des repères visuels, par exemple des termes comme « Pᵢₙₛₚᵢ », « Pₑₓₚᵢ », « Pₘₐₓ », « Pₘᵢₙ » ou « Pᵢₙₛₜ » ou équivalents ou analogues.
- il comprend par ailleurs un ou plusieurs capteurs de débit reliés électriquement à l'unité de commande électronique.
- il comprend une coque ou carcasse rigide portant l'IGU, i.e. l'IGU affleure à la surface d'une des faces de la carcasse du ventilateur.
- la micro-soufflante, le circuit de gaz interne, ledit au moins un capteur de pression et l'unité de commande électronique sont agencés à l'intérieur de la carcasse rigide.
- le circuit de gaz interne est en communication fluidique avec une interface respiratoire patient, tel un masque respiratoire ou une sonde trachéale, de préférence par l'intermédiaire d'un conduit de gaz flexible, aussi appelé tuyau flexible.
- un ou plusieurs capteurs de pression ou de débit sont configurés pour opérer une ou des mesures dans le circuit de gaz interne et/ou dans ou à proximité de l'interface respiratoire patient et/ou dans le conduit de gaz flexible.
- il comprend une interface homme-machine ou IHM servant au personnel soignant à activer ou désactiver une ou des fonctions du ventilateur, par exemple choix de mode ventilatoire ou la catégorie patient (adulte, enfant, nourrisson), et/ou à entrer, sélectionner ou à valider des valeurs ou autres de paramètres ventilatoires, par exemple des valeurs de pression du gaz que le ventilateur médical doit délivrer.
- l'IHM comprend un ou plusieurs boutons, touches, curseurs ou autres.
- l'IHM permet d'entrer ou sélectionner les valeurs désirées de pression d'aide inspiratoire (AI) et de pression expiratoire positive (PEP).
- il comprend en outre une source de courant électrique, c'est-à-dire des moyens d'alimentation électrique, par exemple un câble avec prise secteur ou une ou plusieurs batteries de stockage d'énergie électrique, de préférence rechargeables.
- la source de courant électrique alimente en courant électrique les différents éléments du ventilateur ayant besoin de courant électrique pour fonctionner, notamment la carte électronique, l'afficheur numérique, les capteurs, la micro-soufflante, l'IHM...
- la micro-soufflante alimente le circuit de gaz interne avec de l'air.
- une source d'oxygène, typiquement une bouteille d'oxygène, est reliée au circuit de gaz interne de manière à l'alimenter en oxygène et y réaliser un mélange air/oxygène.

L'appareil de ventilation est particulièrement bien adapté à une utilisation en milieu hospitalier, dans une unité d'urgence ou autre, pour fournir un gaz respiratoire, tel de l'air enrichi ou non en oxygène, à un patient souffrant de troubles respiratoires.

Plus généralement, le fait d'afficher, selon la présente invention, les différents niveaux et/ou valeurs de pression (Pₘₐₓ, Pₘᵢₙ, Pₑₓₚᵢ, Pᵢₙₛₚᵢ et Pᵢₙₛₜ) sur un seul et même graphique, c'est-à-dire sur un graphique unique de type barre-graphe, permet à un personnel soignant de visualiser immédiatement, facilement, et en temps réel, la pression courante patient, c'est-à-dire la pression instantanée (Pᵢₙₛₚᵢ), et de surveiller notamment les seuils de pression maximale (Pₘₐₓ) et pression minimale (Pₘᵢₙ), c'est-à-dire de pouvoir visualiser immédiatement le passage de la pression instantanée (Pᵢₙₛₚᵢ) au-dessus de la pression maximale (Pₘₐₓ) ou, à l'inverse, en-dessous de la pression minimale (Pₘᵢₙ). Ceci constitue alors une aide précieuse pour le personnel soignant pour lui permettre notamment d'éviter tout barotraumatisme du poumon chez le patient, en particulier si des alertes sonores et/ou visuelles, déclenchées par le ventilateur, sont associées à ces seuils de pression par exemple.

En outre, le barre-graphe unique selon l'invention est beaucoup plus facile à interpréter par le personnel soignant que plusieurs courbes de débit et pression comme celles s'affichant sur les ventilateurs usuels, qui requièrent d'être corrélées entre elles, ce qui peut être source d'erreur. Cette interprétation rapide grâce à l'invention est un réel avantage dans l'environnent d'urgence où il faut prendre des décisions rapidement dans un contexte stressant et/ou les personnels soignants qui interviennent en premier lieu, par exemple les pompiers, ont parfois moins de connaissances médicales que ceux en milieux hospitalier, c'est-à-dire les médecins ou analogues.

De plus, les dimensions du barre-graphe peuvent être réduites par rapport à plusieurs courbes, donc il peut être affiché même sur un petit écran équipant le ventilateur, lequel ne serait pas adapté à un affichage de plusieurs courbes simultanément les unes aux autres.

Avantageusement, on prévoit que le barre-graphe peut être conçu pour permettre une adaptation automatique de son échelle en cas de changement de configuration patient, par exemple lors d'une augmentation significative de la valeur de l'AI, de sorte que le personnel soignant ait toujours des informations pertinentes, adaptées et visibles à tout moment de la ventilation du patient.

En d'autres termes, selon la présente invention, afficher toutes les informations de pression utiles sur un même et unique graphe-barre, c'est-à-dire un graphique commun, permet une compréhension plus facile de l'état du patient tout au long de la ventilation mise en œuvre avec détection visuelle facile et rapide de tout passage au-delà des valeurs de pression maximale et/ou minimale.

En particulier, le monitorage, i.e. le suivi, par le personnel soignant, sur un barre-graphe unique ou analogue, de la pression inspiratoire instantanée (Pᵢₙₛₚᵢ) qui varie pendant les phases respiratoires (i.e. valeur et/ou niveau de pression) par rapport aux seuils (i.e. valeurs et/ou niveaux de pression), et de la pression maximale (Pₘₐₓ) permet d'éviter les barotraumas pulmonaires, et par ailleurs de la pression minimale (Pₘᵢₙ) permet de savoir si la pression réglée dans le ventilateur et délivrée par la micro-soufflante arrive bien jusqu'au patient, en évitant ainsi tout risque d'hypoxie qui pourrait se traduire par une hypercapnie, et mettrait en danger la vie du patient.

En cas de dépassement des seuils de pression désirés, une (des) alarme visuelle et/ou sonore peut être déclenchée.

Comme déjà dit, le barre-graphe unique selon l'invention permet au personnel soignant de vérifier rapidement et instantanément, c'est-à-dire en jetant un coup d'œil rapide à l'écran où s'affiche ce graphique avec les différents niveaux de pression, si le patient respire (i.e. affichage des valeurs de pression à l'inspiration et à l'expiration), en confirmant ainsi au personnel soignant l'efficacité du traitement dispensé au moyen du ventilateur.

L'invention va maintenant être mieux comprise grâce à la description détaillée suivante, faite à titre illustratif mais non limitatif, en référence aux figures annexées parmi lesquelles :
Fig. 1 schématise un mode de réalisation d'un appareil de ventilation selon l'invention,
Fig. 2 montre le barre-graphe de la Fig. 3 représenté en parallèle d'une courbe montrant les fluctuations de pression au fil du temps et
Fig. 3 schématise un mode de réalisation d'un graphique de type barre-graphe selon l'invention.

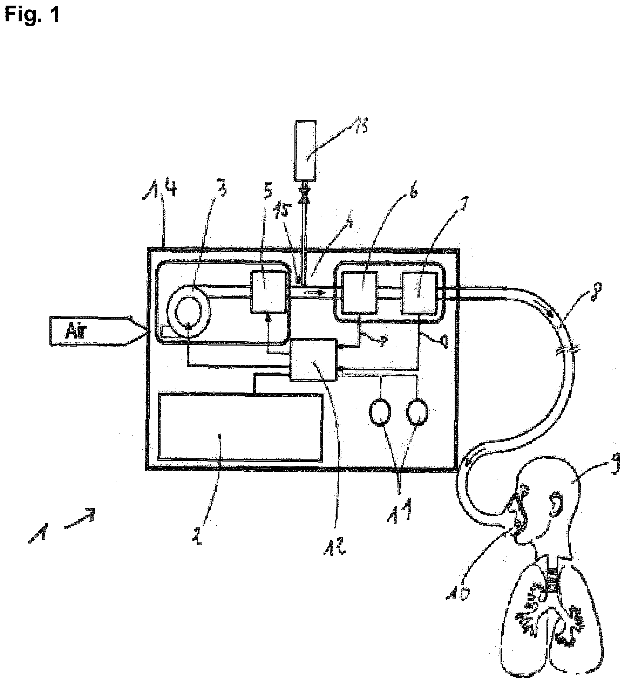

Fig. 1 schématise une architecture d'un mode de réalisation d'un appareil de ventilation 1, c'est-à-dire d'un ventilateur médical d'assistance respiratoire, selon l'invention, lequel est conçu pour fournir un gaz respiratoire à un patient 9, par exemple de l'air ou de l'air enrichi en oxygène provenant d'une source d'oxygène 13, par exemple une bouteille d'oxygène de qualité médicale.

Cet appareil 1 comprend une micro-soufflante 3 motorisée, aussi appelée turbine ou compresseur, délivrant un gaz respiratoire, typiquement de l'air, qui est récupéré et convoyé par un circuit de gaz interne 4, typiquement un ou plusieurs passages ou conduits de gaz aménagés dans la carcasse 14 du ventilateur 1.

Le circuit de gaz interne 4 achemine le gaz respiratoire jusqu'à un conduit ou tuyau flexible 8 en communication fluidique, en amont, avec le circuit de gaz interne 4 et, en aval, avec le patient 9 par l'intermédiaire d'une interface respiratoire 10, tel un masque respiratoire ou analogue. Le conduit flexible 8 vient se raccorder, de manière fluidique et détachable, au ventilateur 1.

Un (ou des) capteur de pression 6 est agencé dans le ventilateur 1 de manière à mesurer la pression du gaz respiratoire dans le circuit de gaz interne 4, c'est-à-dire en aval de la micro-soufflante 3. La mesure de la pression se fait préférentiellement de manière continue.

Le ventilateur 1 comprend en outre une IGU 2 ou afficheur graphique, tel un écran numérique (i.e., digital), de préférence tactile et en couleurs, ainsi qu'une unité de commande électronique 12 comprenant une carte électronique à microprocesseur, de préférence à microcontrôleur, reliée électriquement notamment à la micro-soufflante 3, au capteur de pression 6 et à l'afficheur graphique 2.

Les signaux de pression mesurés par le capteur de pression 6 sont transmis par celui-ci à l'unité de commande électronique 12 faisant office de moyens de pilotage du ventilateur 1, où ils sont traités et convertis en valeurs de pression P, comme détaillé ci-après.

Le ventilateur 1 comprend par ailleurs un premier 5 et un deuxième 7 capteurs de débit (Q) agencés de part et d'autre du capteur de pression 6 et servant à déterminer le débit du flux gazeux, en amont et en aval du site 15 d'injection d'oxygène additionnel éventuel, fourni par la source d'oxygène 13, lorsqu'il y en a une, c'est-à-dire quand un mélange air/O₂ doit être réalisé dans le ventilateur 1 puis fourni au patient 9.

L'unité de commande électronique 12 pilote la micro-soufflante 3 motorisé, en particulier les accélérations et décélérations (i.e. freinage) de son moteur électrique, pour fournir le gaz respiratoire à débit et pression désirés, de préférence sélectionnés par l'utilisateur via l'IHM 11, comme expliqué ci-après.

Ensuite, l'unité de commande électronique 12 à microprocesseur, avantageusement un microcontrôleur mettant en œuvre un ou des algorithmes, monitore et régule la ventilation délivrée au patient 9 en exploitant, i.e. traitant, les signaux de pression et de débit mesurés par les capteurs de débit et/ou de pression 5, 6 et 7.

Les valeurs de pression et/ou de débit peuvent être mémorisés au sein de l'unité de commande électronique 12, notamment au sein d'une mémoire type mémoire flash ou autre, ou dans tout autre moyen de mémorisation, y compris dans un algorithme du microprocesseur.

Une source de courant électrique (non représentée), par exemple une (ou des) batterie(s) intégrée(s) au ventilateur 1 ou un câble avec prise secteur, ou les deux, permet d'alimenter les composants du ventilateur 1 en énergie électrique, notamment la micro-soufflante 3, la carte électronique et/ou le processeur 12, les capteurs de pression 6 et/ou de débit 5, 7, l'afficheur graphique 2, etc...

Par ailleurs, une interface homme-machine 11 (IHM) sert d'interface de réglage ou de sélection, par exemple par action digitale d'un utilisateur, i.e. le personnel soignant, sur des touches de sélection virtuelles et/ou réelles, en particulier des touches virtuelles s'affichant sur l'écran digital de l'IGU 2, typiquement sur un écran tactile. L'IHM 11 peut aussi comprendre de touches de sélection réelles, typiquement des boutons, curseurs ou analogues, permettant le choix et/ou réglage notamment des niveaux de pression souhaités.

L'IHM 11 permet à un utilisateur de fixer ou sélectionner des valeurs de pression d'aide inspiratoire (AI) et de pression expiratoire positive (PEP).

De préférence, la valeur de PEP est choisie entre 0 et 50 cmH₂O, de préférence entre 0 et 30 cmH₂O, typiquement entre 0 et 16 cmH₂O, alors que la valeur d'aide inspiratoire (AI) est choisie entre 1 et 50 cmH₂O, de préférence entre 2 et 35 cm H₂O, typiquement entre 4 et 26 cmH₂O.

A partir de ces valeurs AI et PEP, l'unité de commande électronique 12 est configurée pour calculer ou déterminer une pression maximale (Pₘₐₓ) et une pression minimale (Pₘᵢₙ) telles que :
- Pₘₐₓ = AI + PEP + k, où k est une valeur de pression non-nulle préfixée comprise entre 1 et 20 cm H₂O, de préférence entre 5 et 15 cmH₂O, par exemple k = 10 cmH₂O, et
- 1 cmH₂O < Pₘᵢₙ < 10 cmH₂O, avec Pₘᵢₙ < AI+PEP. Par exemple, la pression minimale (Pₘᵢₙ) est fixée par le ventilateur 1 à :
   ▪ 1 cmH₂O lorsque : AI + PEP < 4 cmH₂O,
   ▪ 4 cmH₂O lorsque : 4 cmH₂O ≤ AI + PEP ≤ 14 cmH₂O, et
   ▪ 10 cmH₂O lorsque : AI + PEP > 14 cmH₂O.

Les valeurs de pression minimale (Pₘᵢₙ) qui sont fonction des valeurs d'AI et de PEP sont mémorisés par le ventilateur 1. Elles peuvent éventuellement être modifiables par l'utilisateur.

Par ailleurs, l'unité de commande électronique 12 du ventilateur 1 est configurée pour calculer ou déterminer, à partir des signaux de mesure de pression provenant du capteur de pression 6, au moins des valeurs de pression expiratoire (Pₑₓₚᵢ), de pression inspiratoire (Pᵢₙₛₚ) et de pression instantanée (Pᵢₙₛₜ), sachant que :
- la valeur de pression expiratoire (Pₑₓₚᵢ) du patient considérée correspondant à la valeur de pression mesurée à la fin de chaque phase expiratoire du patient,
- la valeur de pression inspiratoire (Pᵢₙₛₚ) du patient considérée correspondant à la valeur de pression mesurée à la fin de chaque phase inspiratoire du patient,
- et la pression instantanée (Pᵢₙₛₜ) correspondant à la valeur de pression instantanée mesurée en continu.

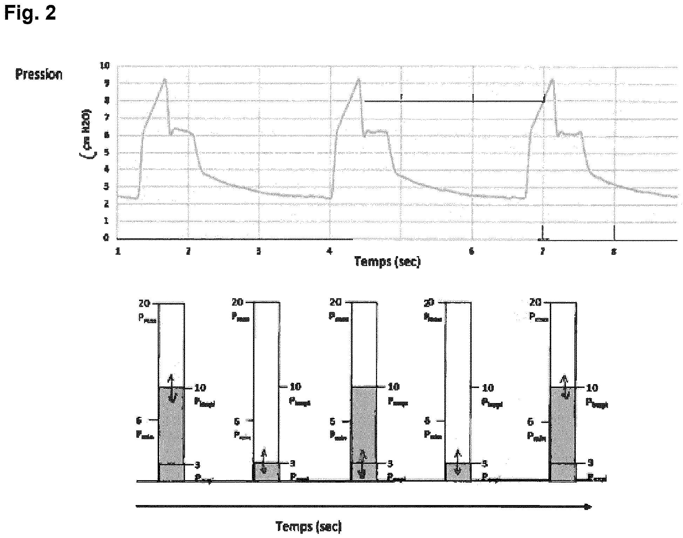

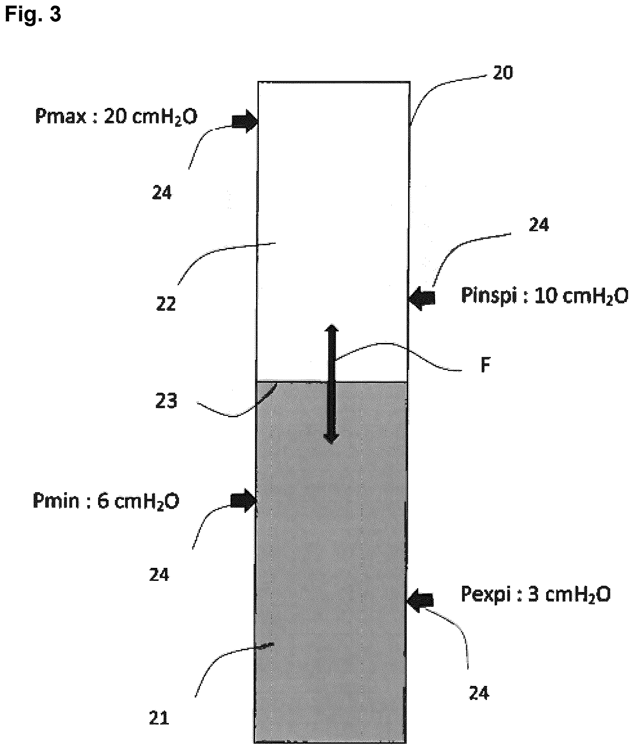

Selon l'invention, afin d'assister le personnel soignant dans sa surveillance du patient 9 et dès lors d'éviter un barotraumatisme des poumons ou, à l'inverse, une hypoxie du patient 9, l'IGU 2 est conçue et configurée pour afficher graphiquement et simultanément sur un seul graphique unique 20, tel un barre-graphe, l'ensemble des niveaux et/ou valeurs de pression utiles au personnel soignant, comme illustré en Fig. 2 ou Fig. 3, à savoir les valeurs ou niveaux de pression maximale (Pₘₐₓ), pression minimale (Pₘᵢₙ), pression expiratoire (Pₑₓₚᵢ), pression inspiratoire (Pᵢₙₛₚ) et pression instantanée (Pᵢₙₛₜ).

La pression inspiratoire instantanée (Pᵢₙₛₜ) fluctue au fil du temps, comme illustré par la flèche F sur la Fig. 3.

Après traitement des signaux des mesures de pressions opérées par le capteur de pression 6, le microprocesseur de l'unité de commande électronique 12 peut déterminer une (ou des) valeur de pression instantanée (Pᵢₙₛₜ) à un instant donné (t) ou à plusieurs (n) instants (t) successifs (t₀, t₁, t₂...tₙ), en particulier sur une durée correspondant à un ou plusieurs cycles de ventilation, ainsi que les pressions inspiratoire (Pᵢₙₛₚ) et expiratoire (Pₑₓₚᵢ) à chaque cycle respiratoire (i.e. phase expiratoire et phase inspiratoire).

Le microprocesseur de l'unité de commande électronique 12 transmet ensuite les différentes valeurs de pression (Pᵢₙₛₚᵢ, Pₑₓₚᵢ, Pᵢₙₛₜ, Pₘₐₓ et Pₘᵢₙ) à l'IGU 2 en vue de leur affichage simultané sur l'écran digital de l'IGU 2, sur un graphique 20 commun et unique selon l'invention, tel le barre-graphe visible en Fig. 3 où les indications Pᵢₙₛₚᵢ, Pₑₓₚᵢ, Pₘₐₓ et Pₘᵢₙ sont affichées de préférence en regard des valeurs correspondantes, comme illustré en Fig. 2. Le niveau de pression instantanée Pᵢₙₛₜ est affiché quant à lui sous forme d'un repère ou indicateur mobile 23 (i.e. hauteur de la zone grisée 21), comme expliqué ci-après.

Optionnellement, la valeur de PEP peut aussi être affichée sur l'écran digital de l'IGU 2. De même, optionnellement, on peut aussi décider d'afficher la valeur de pression d'aide inspiratoire (AI) sur le graphique.

Avantageusement, le microprocesseur de l'unité de commande électronique 12 peut déclencher une alarme sonore et visuelle pour avertir l'utilisateur de situations dangereuses en comparant la ou les pressions Pᵢₙₛₚᵢ et/ou Pₑₓₚᵢ, aux seuils de pressions Pₘₐₓ et Pₘᵢₙ, où :
- Pₘₐₓ peut être définie comme la pression que Pᵢₙₛₚᵢ ne doit pas dépasser car synonyme de danger pour le patient (e.g. barotraumatisme...).
- Pₘᵢₙ peut être définie comme la pression minimale qui doit être maintenue dans le chemin de gaz du ventilateur 1. Si la Pᵢₙₛₚᵢ devient inférieure à Pₘᵢₙ, alors : soit le patient 9 est déconnecté du ventilateur 1, par exemple masque retiré, soit il existe un défaut empêchant la délivrance du gaz, tel exemple un pincement du conduit flexible 8.

Comme illustré en Fig. 3, le graphique 20 comprend un barre-graphe comprenant une zone grisée 21 (i.e. colorée) et zone blanche 22 permettant de visualiser les évolutions ou fluctuations (flèche F) de l'indicateur mobile 23, séparant la zone blanche 22 de la zone grisée 21, lequel est représentatif du niveau de pression instantanée (Pᵢₙₛₚᵢ) et ce, par rapport aux indicateurs 24 correspondant aux différentes pressions désirées, à savoir Pₑₓₚᵢ, Pᵢₙₛₚᵢ, Pₘₐₓ et Pₘᵢₙ.

Plus précisément :
- Les valeurs de Pₘₐₓ et de Pₘᵢₙ et les indicateurs 24 correspondants sont fixes, c'est-à-dire qu'elles ne varient pas d'un cycle respiratoire à un autre.
- Les valeurs de Pₑₓₚᵢ et de Pᵢₙₛₚᵢ et les indicateurs 24 correspondants varient ou non selon les cycles respiratoires.
- La zone grisée 21 et l'indicateur mobile 23 varient (quasi) en permanence puisque la pression instantanée fluctue en continu.

La zone grisée 21 s'étend entre le bas du barre-graphe 20, qui a ici de forme de rectangle vertical allongé, et un repère visuel 23, qui est ici une barre horizontale représentatif de la valeur de pression instantanée (Pᵢₙₛₚᵢ) mesurée qui fluctue pendant les cycles respiratoires.

En effet, au fil des cycles respiratoires, le repère visuel 23 de pression instantanée (Pᵢₙₛₜ) est mobile verticalement, comme montré par la flèche F, ce qui augmente ou diminue la surface de la zone grisée 21, et fournit alors une image de l'évolution instantanée de la pression délivrée au patient 9. En d'autres termes, la hauteur de la surface ou zone grisée 21 est proportionnelle à la valeur Pᵢₙₛₜ et fluctue au fil du temps, dans le sens vertical (i.e. de bas en haut et inversement) indiqué par la flèche F.

Préférentiellement, des caractères alphanumériques (texte, lettres, chiffres ou autres) sont affichés en regard des repères visuels 24 pour indiquer à l'utilisateur la nature de la valeur de pression affichée.

Par exemple, les repères 24 sont des traits, des petites flèches (comme sur la Fig. 3, des points ou tout autre forme. Par ailleurs, les valeurs numériques des pressions Pₑₓₚᵢ, Pᵢₙₛₚᵢ, Pₘₐₓ et Pₘᵢₙ peuvent aussi être affichées en regard des dénominations des différentes pressions (Pₑₓₚᵢ, Pᵢₙₛₚᵢ, Pₘₐₓ et Pₘᵢₙ), c'est-à-dire à côté ou à proximité par exemple, des repères visuels 24, comme illustré en Fig. 2.

Les caractères ou textes et les repères visuels 24 peuvent être avantageusement affichés sur l'écran digital de l'IGU 2.

Ainsi, dans l'exemple présenté en Fig. 2 et Fig. 3, les valeurs numériques de Pₘₐₓ (20 cmH₂O), de Pₘᵢₙ (6 cmH₂O), de Pₑₓₚᵢ (3 cmH₂O) et de Pᵢₙₛₚᵢ (10 cmH₂O) sont affichées en regard du barre-graphe 20.

Ceci permet de renseigner immédiatement l'utilisateur qui peut se rendre compte de la qualité de la ventilation en jetant un coup d'œil rapide au graphique 20, sans avoir à interpréter plusieurs courbes ou analogues, comme c'est le cas avec les appareils de l'art antérieur.

Ainsi, l'utilisateur, i.e. personnel soignant, peut savoir instantanément si la pression inspiratoire instantanée Pᵢₙₛₚᵢ délivrée au patient 9 à un instant donné, varie ou non entre les limites de pression haute et basse, notamment Pₘₐₓ et Pₘᵢₙ, entre lesquelles elles doivent évoluer pour ne représenter aucun danger ventilatoire pour le patient 9. De même, il peut avoir un historique des cycles ventilatoires précédents grâce à un affichage sur le même graphique 20 des valeurs de pression Pᵢₙₛₚᵢ et Pₑₓₚᵢ obtenues sur un ou préférentiellement plusieurs cycles respiratoires précédents.

Les indications ou repères visuels 24 et/ou les valeurs de pression et/ou les textes éventuels, telles les dénominations des différentes pressions d'intérêt, peuvent être affichées sur l'écran de l'IGU 2 sur ou à côté du barre-graphe 20 et évoluer en fonction du mode de ventilation ou du statut du ventilateur (i.e. en attente, arrêt, ventilation...).

Autrement dit, les valeurs des pressions peuvent être affichées sur un écran dédié ou sur un écran commun de l'IGU 2. Les valeurs affichées sont mises à jour en fonction de la ventilation délivrée et des pressions mesurées.

Si la pression instantanée (Pᵢₙₛₜ) qui est représentée par l'indicateur visuel 23 dépasse un seuil donné, alors le seuil dépassé et/ou l'indicateur visuel 23 peuvent changer de couleur pour en informer l'utilisateur. Une alarme sonore peut aussi être déclenchée simultanément.

L'utilisateur peut par ailleurs modifier un seuil de pression par exemple en agissant sur les moyens de réglage, par exemple en interagissant avec un bouton physique de l'IHM 11 du ventilateur 1, en choisissant un nouveau seuil par manipulation tactile de l'écran de l'IGU 2 dans le cas d'une IHM tactile s'affichant sur ledit écran d'affichage 2.

Fig. 2 représente en parallèle les évolutions d'une courbe de pression au fil du temps, c'est-à-dire des fluctuations de pression (en cmH₂O) pendant plusieurs cycles respiratoires, de manière à mieux comprendre ce qui s'affiche sur le graphique unique 20 selon l'invention en fonction des évolutions de la pression délivrée au patient 9.

Comme on le voit, l'écran de l'IGU 2 du ventilateur 1 est configurée pour afficher, sur le barre-graphe unique 20 (partie basse de la Fig. 2, les pressions maximale Pₘₐₓ et minimale Pₘᵢₙ préfixées, ainsi que la pression inspiratoire Pᵢₙₛₚᵢ et la pression expiratoire Pₑₓₚᵢ atteinte sur ici trois cycles ventilatoires successifs (visible sur la courbe du haut de la Fig. 2).

La pression instantanée Pᵢₙₛₜ est représentée quant à elle par la hauteur de la partie grisée 22 du barre-graphe 20 comme déjà expliqué (cf. Fig. 3), laquelle fluctue selon le sens de la flèche F au cours des cycles respiratoires.

Avantageusement, les valeurs de pressions maximales associées aux dénominations des différentes pressions sont affichées en une couleur attirant l'attention, par exemple en rouge ou orange, alors que les autres niveaux et/ou valeurs de pressions associées peuvent être affichées une couleur plus discrète, par exemple en noir, en gris, en vert ou en bleu par exemple.

Dans l'exemple présenté, on constate que, pendant ces cycles ventilatoires, la pression expiratoire (Pₑₓₚᵢ) atteinte a été de l'ordre de 3 cmH₂0 et que, par ailleurs, la pression instantanée Pinst a fluctué, selon le moment considéré, entre 3 et 10 cmH₂O, comme le montre la hauteur de la zone grisée 21, c'est-à-dire la position verticale de l'indicateur 23, et ce, pour une pression maximale (Pₘₐₓ) fixée à 20 cmH₂0 et une pression minimale (Pₘᵢₙ) fixée à 6 cm H₂0.

On comprend en regardant les graphiques 20 schématisés aux différents moments des cycles de la Fig. 2, que le personnel soignant peut immédiatement avoir une bonne idée de la ventilation dispensée au patient 9 grâce à une visualisation aisée et instantanée de toutes les pressions P importantes à suivre pour s'assurer que la ventilation se déroule dans de bonnes conditions, lesquelles s'affichent sur un même barre-graphe 20.

Le personnel soignant n'a donc plus à réfléchir pour réaliser une corrélation entre les différents paramètres affichés puisque la corrélation est opérée par le ventilateur 1 lui-même, ce qui permet de minimiser les risques d'erreur et donc de mauvaise ventilation du patient et donc améliorer les soins octroyés aux patients.

## Revendications

1. Appareil de ventilation (1) pour fournir un gaz respiratoire à un patient (9) comprenant une micro-soufflante (3) délivrant un gaz respiratoire, un circuit de gaz interne (4) pour convoyer le gaz respiratoire, au moins un capteur de pression (6) pour mesurer la pression du gaz, une interface graphique utilisateur (2) et une unité de commande électronique (12) reliée électriquement à la micro-soufflante (3), au capteur de pression (6) et à l'interface graphique utilisateur (2), ledit capteur de pression (6) étant par ailleurs configuré pour mesurer et transmettre à l'unité de commande électronique (12), des signaux de mesure de pression, **caractérisé en ce que** :
- il comprend des moyens de réglage (11) configurés pour permettre à un utilisateur de fixer ou sélectionner :
∘ au moins une valeur de pression d'aide inspiratoire (AI) comprise entre 1 et 50 cmH₂O et
∘ au moins une valeur de pression expiratoire positive (PEP) comprise entre 0 et 50 cmH₂O,
- l'unité de commande électronique (12) est configurée pour calculer, fixer, sélectionner ou déterminer:
▪ une pression maximale (Pₘₐₓ) à partir des valeurs de pression d'aide inspiratoire (AI) et de pression expiratoire positive (PEP) fixées ou sélectionnées par l'utilisateur, avec Pₘₐₓ = AI + PEP + k, où k est une valeur de pression non-nulle préfixée comprise entre 1 et 20 cm H₂O, et
▪ une pression minimale (Pₘᵢₙ) comprise entre 1 et 10 cm H₂O, avec Pₘᵢₙ < AI+PEP,
- l'unité de commande électronique (12) est configurée pour calculer ou déterminer, à partir des signaux de mesure de pression provenant du capteur de pression (6), au moins :
o une pression expiratoire (Pₑₓₚᵢ) du patient correspondant à la valeur de pression mesurée à la fin de chaque phase expiratoire du patient,
o une pression inspiratoire (Pᵢₙₛₚ) du patient correspondant à la valeur de pression mesurée à la fin de chaque phase inspiratoire du patient et
une pression instantanée (Pᵢₙₛₜ) correspondant à la valeur de pression instantanée mesurée en continu,
- et l'interface graphique utilisateur (2) est configurée pour afficher simultanément sur un graphique unique (20), au moins :
∘ la pression maximale (Pₘₐₓ),
∘ la pression minimale (Pₘᵢₙ),
∘ la pression expiratoire (Pₑₓₚᵢ),
∘ la pression inspiratoire (Pᵢₙₛₚ) et
∘ la pression instantanée (Pᵢₙₛₜ).

2. Appareil de ventilation selon la revendication 1, **caractérisé en ce que** l'unité de commande électronique (12) est configurée pour fixer la pression minimale (Pₘᵢₙ) à:
- 1 cmH₂O lorsque AI + PEP < 4 cmH₂O,
- 4 cmH₂O lorsque 4 cmH₂O ≤ AI + PEP ≤ 14 cmH₂O, et
- 10 cmH₂O lorsque AI + PEP > 14 cmH₂O.

3. Appareil selon l'une des revendications 1 ou 2, **caractérisé en ce que** l'unité de commande électronique (12) est configurée pour calculer, fixer, sélectionner ou déterminer une pression maximale (Pₘₐₓ) à partir des valeurs de pression d'aide inspiratoire (AI) et de pression expiratoire positive (PEP) fixées ou sélectionnées par l'utilisateur, avec Pₘₐₓ = AI + PEP + k, où la valeur de pression k est comprise entre 5 et 15 cmH₂O, de préférence égale à 10 cmH₂O.

4. Appareil selon l'une des revendications 1 à 3, **caractérisé en ce que** l'unité de commande électronique (12) est configurée pour calculer, fixer, sélectionner ou déterminer une pression maximale (Pₘₐₓ) à partir des valeurs de pression d'aide inspiratoire (AI) et de pression expiratoire positive (PEP) fixées ou sélectionnées par l'utilisateur, avec Pₘₐₓ = AI + PEP + k, où la valeur de PEP est comprise entre 0 et 30 cmH₂O, de préférence entre 0 et 16 cmH₂O.

5. Appareil selon l'une des revendications 1 à 4, **caractérisé en ce que** l'unité de commande électronique (12) est configurée pour calculer, fixer, sélectionner ou déterminer une pression maximale (Pₘₐₓ) à partir des valeurs de pression d'aide inspiratoire (AI) et de pression expiratoire positive (PEP) fixées ou sélectionnées par l'utilisateur, avec Pₘₐₓ = AI + PEP + k, où la valeur d'aide inspiratoire (AI) est comprise entre 2 et 35 cm H₂O, de préférence entre 4 et 26 cmH₂O.

6. Appareil selon la revendication 1, **caractérisé en ce que** l'IGU (2) comprend un écran d'affichage, de préférence un écran numérique.

7. Appareil de ventilation selon l'une des revendications 1 ou 6, **caractérisé en ce que** l'IGU (2) est configurée pour afficher les niveaux et/ou valeurs de pression sur un graphique unique représentant un barre-graphe.

8. Appareil de ventilation selon l'une des revendications 1, 6 ou 7, **caractérisé en ce que** l'IGU (2) est configurée pour afficher des repères visuels (22, 23) représentant des niveaux de pression.

9. Appareil de ventilation selon l'une des revendications 1 ou 6 à 8, **caractérisé en ce que** l'IGU (2) est configurée pour afficher des caractères ou textes identifiant la nature des repères visuels (22).

10. Appareil de ventilation selon l'une des revendications 1 ou 6 à 9, **caractérisé en ce que** l'IGU (2) est configurée pour afficher des valeurs numériques de pressions en regard des repères visuels (22).

## Patentansprüche

1. Beatmungsgerät (1) zum Liefern eines Atemgases an einen Patienten (9), das ein ein Atemgas lieferndes Mikrogebläse (3), einen inneren Gaskreislauf (4) zum Befördern des Atemgases, mindestens einen Drucksensor (6) zur Messung des Drucks des Gases, eine graphische Benutzerschnittstelle (2) und eine elektronische Steuereinheit (12) enthält, die elektrisch mit dem Mikrogebläse (3), dem Drucksensor (6) und der graphischen Benutzerschnittstelle (2) verbunden ist, wobei der Drucksensor (6) außerdem konfiguriert ist, Druckmesssignale zu messen und an die elektronische Steuereinheit (12) zu übertragen, **dadurch gekennzeichnet, dass**:
- es Einstelleinrichtungen (11) enthält, die konfiguriert sind, einem Benutzer zu ermöglichen, festzulegen oder auszuwählen:
∘ mindestens einen Einatemunterstützungsdruckwert (AI) zwischen 1 und 50 cmH₂O und
∘ mindestens einen positiven Ausatemdruckwert (PEP) zwischen 0 und 50 cmH₂O,
- die elektronische Steuereinheit (12) konfiguriert ist, zu berechnen, festzulegen, auszuwählen oder zu bestimmen:
• einen maximalen Druck (Pₘₐₓ) ausgehend von den vom Benutzer festgelegten oder ausgewählten Werten des Einatemunterstützungsdrucks (AI) und des positiven Ausatemdrucks (PEP), mit Pₘₐₓ = AI + PEP + k, wobei k ein vorab festgelegter Druckwert ungleich Null zwischen 1 und 20 cmH₂O ist, und
• einen minimalen Druck (Pₘᵢₙ) zwischen 1 und 10 cmH₂O, mit Pₘᵢₙ < AI+PEP,
- die elektronische Steuereinheit (12) konfiguriert ist, ausgehend von den vom Drucksensor (6) kommenden Druckmesssignalen mindestens zu berechnen oder zu bestimmen:
∘ einen Ausatemdruck (Pₑₓₚᵢ) des Patienten entsprechend dem am Ende jeder Ausatmungsphase des Patienten gemessenen Druckwert,
∘ einen Einatemdruck (Pᵢₙₛₚ) des Patienten entsprechend dem am Ende jeder Einatmungsphase des Patienten gemessenen Druckwert, und
∘ einen Augenblicksdruck (Pᵢₙₛₜ) entsprechend dem fortlaufend gemessenen Augenblicksdruckwert,
- und die graphische Benutzerschnittstelle (2) konfiguriert ist, gleichzeitig auf einer einzigen Grafik (20) mindestens anzuzeigen:
∘ den maximalen Druck (Pₘₐₓ),
∘ den minimalen Druck (Pₘᵢₙ),
∘ den Ausatemdruck (Pₑₓₚᵢ),
∘ den Einatemdruck (Pᵢₙₛₚ) und
∘ den Augenblicksdruck (Pᵢₙₛₜ).

2. Beatmungsgerät nach Anspruch 1, **dadurch gekennzeichnet, dass** die elektronische Steuereinheit (12) konfiguriert ist, den minimalen Druck (Pₘᵢₙ) festzulegen auf:
- 1 cmH₂O wenn AI + PEP < 4 cmH₂O,
- 4 cmH₂O wenn 4 cmH₂O ≤ AI + PEP ≤ 14 cmH₂O, und
- 10 cmH₂O wenn AI + PEP > 14 cmH₂O.

3. Gerät nach einem der Ansprüche 1 oder 2, **dadurch gekennzeichnet, dass** die elektronische Steuereinheit (12) konfiguriert ist, einen maximalen Druck (Pₘₐₓ) ausgehend von den vom Benutzer festgelegten oder ausgewählten Werten des Einatemunterstützungsdrucks (AI) und des positiven Ausatemdrucks (PEP) zu berechnen, festzulegen, auszuwählen oder zu bestimmen, mit Pₘₐₓ = AI + PEP + k, wobei der Druckwert k zwischen 5 und 15 cmH₂O liegt, vorzugsweise gleich 10 cmH₂O ist.

4. Gerät nach einem der Ansprüche 1 bis 3, **dadurch gekennzeichnet, dass** die elektronische Steuereinheit (12) konfiguriert ist, einen maximalen Druck (Pₘₐₓ) ausgehend von den vom Benutzer festgelegten oder ausgewählten Werten des Einatemunterstützungsdrucks (AI) und des positiven Ausatemdrucks (PEP) zu berechnen, festzulegen, auszuwählen oder zu bestimmen, mit Pₘₐₓ = AI + PEP + k, wobei der Wert von PEP zwischen 0 und 30 cmH₂O liegt, vorzugsweise zwischen 0 und 16 cmH₂O.

5. Gerät nach einem der Ansprüche 1 bis 4, **dadurch gekennzeichnet, dass** die elektronische Steuereinheit (12) konfiguriert ist, einen maximalen Druck (Pₘₐₓ) ausgehend von den vom Benutzer festgelegten oder ausgewählten Werten des Einatemunterstützungsdrucks (AI) und des positiven Ausatemdrucks (PEP) zu berechnen, festzulegen, auszuwählen oder zu bestimmen, mit Pₘₐₓ = AI + PEP + k, wobei der Einatemunterstützungswert (AI) zwischen 2 und 35 cmH₂O liegt, vorzugsweise zwischen 4 und 26 cmH₂O.

6. Gerät nach Anspruch 1, **dadurch gekennzeichnet, dass** die IGU (2) einen Anzeigebildschirm enthält, vorzugsweise einen digitalen Bildschirm.

7. Beatmungsgerät nach einem der Ansprüche 1 oder 6, **dadurch gekennzeichnet**, die IGU (2) konfiguriert ist, die Druckpegel und/oder -werte auf einer einzigen Grafik anzuzeigen, die eine Balkengrafik darstellt.

8. Beatmungsgerät nach einem der Ansprüche 1, 6 oder 7, **dadurch gekennzeichnet, dass** die IGU (2) konfiguriert ist, visuelle Markierungen (22, 23) anzuzeigen, die Druckpegel darstellen.

9. Beatmungsgerät nach einem der Ansprüche 1 oder 6 bis 8, **dadurch gekennzeichnet, dass** die IGU (2) konfiguriert ist, Buchstaben oder Texte anzuzeigen, die die Art der visuellen Markierungen (22) bestimmen.

10. Beatmungsgerät nach einem der Ansprüche 1 oder 6 bis 9, **dadurch gekennzeichnet, dass** die IGU (2) konfiguriert ist, digitale Werte von Drücken gegenüber den visuellen Markierungen (22) anzuzeigen.

## Claims

1. Ventilation apparatus (1) for providing a respiratory gas to a patient (9), comprising a micro blower (3) delivering a respiratory gas, an internal gas circuit (4) for conveying the respiratory gas, at least one pressure sensor (6) for measuring the pressure of the gas, a graphical user interface (2) and an electronic control unit (12) electrically connected to the micro blower (3), to the pressure sensor (6) and to the graphical user interface (2), said pressure sensor (6) furthermore being configured to measure and transmit pressure measurement signals to the electronic control unit (12), **characterized in that**:
- it comprises adjustment means (11) which are configured to allow a user to set or select:
∘ at least one inhalation aid pressure (AI) value between 1 and 50 cmH₂O and
∘ at least one positive expiratory pressure (PEP) value between 0 and 50 cmH₂O,
- the electronic control unit (12) is configured to calculate, set, select or determine:
▪ a maximum pressure (Pₘₐₓ) on the basis of the inhalation aid pressure (AI) and positive expiratory pressure (PEP) values set or selected by the user, with Pₘₐₓ = AI + PEP + k, where k is a pre-set non-zero pressure value between 1 and 20 cmH₂O, and
▪ a minimum pressure (Pₘᵢₙ) between 1 and 10 cmH₂O, with Pₘᵢₙ < AI + PEP,
- the electronic control unit (12) is configured to calculate or determine, on the basis of the pressure measurement signals originating from the pressure sensor (6), at least:
∘ an expiratory pressure (Pₑₓₚᵢ) of the patient corresponding to the pressure value measured at the end of each expiratory phase of the patient,
∘ an inhalation pressure (Pᵢₙₛₚ) of the patient corresponding to the pressure value measured at the end of each inhalation phase of the patient, and
∘ an instantaneous pressure (Pᵢₙₛₜ) corresponding to the continuously measured instantaneous pressure value,
- and the graphical user interface (2) is configured to simultaneously display, on a single graphical item (20), at least:
∘ the maximum pressure (Pₘₐₓ),
∘ the minimum pressure (Pₘᵢₙ),
∘ the expiratory pressure (Pₑₓₚᵢ),
∘ the inhalation pressure (Pᵢₙₛₚ), and
∘ the instantaneous pressure (Pᵢₙₛₜ).

2. Ventilation apparatus according to Claim 1, **characterized in that** the electronic control unit (12) is configured to set the minimum pressure (Pₘᵢₙ) to:
- 1 cmH₂O when AI + PEP < 4 cmH₂O,
- 4 cmH₂O when 4 cmH₂O ≤ AI + PEP ≤ 14 cmH₂O, and
- 10 cmH₂O when AI + PEP > 14 cmH₂O.

3. Apparatus according to either of Claims 1 and 2, **characterized in that** the electronic control unit (12) is configured to calculate, set, select or determine a maximum pressure (Pₘₐₓ) on the basis of the inhalation aid pressure (AI) and positive expiratory pressure (PEP) values set or selected by the user, with Pₘₐₓ = AI + PEP + k, where the pressure value k is between 5 and 15 cmH₂O, preferably equal to 10 cmH₂O.

4. Apparatus according to one of Claims 1 to 3, **characterized in that** the electronic control unit (12) is configured to calculate, set, select or determine a maximum pressure (Pₘₐₓ) on the basis of the inhalation aid pressure (AI) and positive expiratory pressure (PEP) values set or selected by the user, with Pₘₐₓ = AI + PEP + k, where the value of PEP is between 0 and 30 cmH₂O, preferably between 0 and 16 cmH₂O.

5. Apparatus according to one of Claims 1 to 4, **characterized in that** the electronic control unit (12) is configured to calculate, set, select or determine a maximum pressure (Pₘₐₓ) on the basis of the inhalation aid pressure (AI) and positive expiratory pressure (PEP) values set or selected by the user, with Pₘₐₓ = AI + PEP + k, where the inhalation aid (AI) value is between 2 and 35 cmH₂O, preferably between 4 and 26 cmH₂O.

6. Apparatus according to Claim 1, **characterized in that** the GUI (2) comprises a display screen, preferably a digital screen.

7. Ventilation apparatus according to either of Claims 1 and 6, **characterized in that** the GUI (2) is configured to display the pressure levels and/or values on a single graphical item representing a bar graph.

8. Ventilation apparatus according to one of Claims 1, 6 or 7, **characterized in that** the GUI (2) is configured to display visual markers (22, 23) representing pressure levels.

9. Ventilation apparatus according to one of Claims 1 or 6 to 8, **characterized in that** the GUI (2) is configured to display characters or text identifying the nature of the visual markers (22).

10. Ventilation apparatus according to one of Claims 1 or 6 to 9, **characterized in that** the GUI (2) is configured to display digital pressure values with reference to the visual markers (22).
